Europäisches Patentamt

European Patent Office

Office européen des brevets

(19)

(11) **EP 1 210 308 B1**

(12) **EUROPÄISCHE PATENTSCHRIFT**

(45) Veröffentlichungstag und Bekanntmachung des Hinweises auf die Patenterteilung:
**03.09.2003   Patentblatt 2003/36**

(21) Anmeldenummer: **00944018.1**

(22) Anmeldetag: **08.07.2000**

(51) Int Cl.⁷: $C07C\ 29/151$, $B01J\ 8/04$

(86) Internationale Anmeldenummer:
**PCT/EP00/06488**

(87) Internationale Veröffentlichungsnummer:
**WO 01/017935 (15.03.2001 Gazette 2001/11)**

(54) **VERFAHREN UND ANLAGE ZUR METHANOLSYNTHESE AUS WASSERSTOFF, KOHLENMONOXID UND KOHLENDIOXID UNTER DRUCK**

METHOD AND INSTALLATION FOR SYNTHESISING METHANOL FROM HYDROGEN, CARBON MONOXIDE AND CARBON DIOXIDE UNDER PRESSURE

PROCEDE ET INSTALLATION DE SYNTHESE DU METHANOL A PARTIR D'HYDROGENE, MONOXYDE DE CARBONE ET DIOXYDE DE CARBONE SOUS PRESSION

(84) Benannte Vertragsstaaten:
**AT BE CH CY DE DK ES FI FR GB GR IE IT LI LU MC NL PT SE**

(30) Priorität: **07.09.1999   DE 19942559**
**23.10.1999   DE 19951137**
**07.01.2000   DE 10000280**

(43) Veröffentlichungstag der Anmeldung:
**05.06.2002   Patentblatt 2002/23**

(73) Patentinhaber: **Uhde GmbH**
**44141 Dortmund (DE)**

(72) Erfinder: **BÄHNISCH, Hans-Joachim**
**D-44227 Dortmund (DE)**

(74) Vertreter: **Dabringhaus, Walter, Dipl.-Ing. Patentanwälte**
**Meinke, Dabringhaus und Partner,**
**Rosa-Luxemburg-Strasse 18**
**44141 Dortmund (DE)**

(56) Entgegenhaltungen:
**DE-A- 3 518 362**

## Beschreibung

Die Erfindung richtet sich auf ein Verfahren zur Methanolsynthese aus Wasserstoff, Kohlenmonoxid und Kohlendioxid unter Druck, insbesondere zur Steigerung der Ausbeute bereits eingesetzter Verfahren, wobei entschwefeltes Erdgas einem Reformer und nachfolgend das Synthesegas einer Methanolsynthese aufgegeben wird.

Es sind eine Reihe von Anlagen bzw. Verfahren zur katalytischen Methanolsynthese bekannt, wobei für die Fülle der Lösungen hier als Beispiel die Schriften DE-21 17 060, DE-25 29 591, DE-32 20 995, DE-35 18 362, US-29 04 575 und DE-41 00 632 genannt seien.

Normalerweise wird eine Methanolerzeugungsanlage im Verbund mit einer Anlage zur Erzeugung von Synthesegas betrieben, wobei beide Anlagen so dimensioniert werden, daß das erzeugte Synthesegas die Anforderungen des methanolerzeugenden Katalysators des Synthesegaskreislaufes genau abdeckt und insbesondere stöchiometrisch bezogen auf die folgenden Reaktionen zusammengesetzt ist, wobei nur zwei davon linear abhängig voneinander sind:

$$CO + 2\,H_2 \leftrightarrow CH_3OH - 90,84\ kJ/mol \tag{1}$$

$$CO_2 + H_2 \leftrightarrow CO + H_2O + 41,20\ kJ/mol \tag{2}$$

$$CO_2 + 3\,H_2 \leftrightarrow CH_3OH + H_2O - 49,64\ kJ/mol \tag{3}$$

Nach diesen Reaktionsgleichungen gilt für ein stöchiometrisches Synthesegas:

$$\zeta = \frac{\dot{c_{iH_2}} - \dot{c_{iCO_2}}}{\dot{c_{iCO}} + \dot{c_{iCO_2}}} = 2 \tag{4}$$

wobei $c_i$ Gaskonzentrationen der betreffenden Ausgangsstoffe auf Mol-Basis sind.

Ein solches Synthesegas wird üblicherweise in einem Primärreformer allein oder zusammen mit einem Sekundärreformer oder in ähnlichen Gaserzeugungsanlagen einstraßig hergestellt.

Eine solche Anlage ist nur schwer nachrüstbar, wenn es gilt, bestehende Produktionskapazitäten zu erweitern. Im allgemeinen muß in einem solchen Fall eine weitere Anlage nach dem Muster der Altanlage gebaut werden und Synergieeffekte beider Anlagen sind nicht zu erwarten. Problematisch ist weiterhin die Einbindung von Fremd-Synthesegas, was nach Stillegungen anderer Anlagen in größeren Werken oft billig zur Verfügung steht, aber aufgrund anderer Gaszusammensetzungen, meist aufgrund eines zu großen Anteils kohlenstoffhaltiger Verbindungen ("C-Anteil"), nicht ohne aufwendige, weitere Aufarbeitung zur Methanolerzeugung genutzt werden kann.

Aufgabe der Erfindung ist es daher, das bekannte Verfahren dahingehend zu erweitern, daß die bekanntgewordenen Nachteile überwunden werden, eine günstige Nachrüstbarkeit bestehender Anlagen geschaffen wird und Fremd-Synthesegas eingesetzt werden kann.

Mit einem Verfahren der eingangs bezeichneten Art wird diese Aufgabe gemäß der Erfindung dadurch gelöst, daß nach Durchlaufen des Reformers aus dem Synthesegasstrom ein Seitenstrom einem Methanol-Vorreaktor zugeführt wird, das im Vorreaktor erzeugte Methanol dem die Methanolsynthese des Hauptstromes verlassenden Methanolstrom zugeführt und ein im Methanol-Vorreaktor nicht umgesetzter Synthesegasstrom dem Hauptstrom vor der Methanolsynthese wieder zugeführt wird, wobei im Bereich dieser Zuführung gleichzeitig ein den entstandenen Verlust ausgleichendes zusätzliches Synthesegas aufgegeben wird.

Der zum Einsatz kommende Methanol-Vorreaktor sowie die üblichen Vorrichtungen zum Konditionieren des Synthesegases sowie des Auskondensierens und Abscheidens von erzeugtem Methanol können nachgerüstet werden, wobei eine Vergrößerung der Produktionskapazität von bis zu 57% bezogen auf die Kapazität der Altanlage auf diese Weise erreichbar ist. Natürlich ist dieses Konzept nicht auf die Nachrüstung von Altanlagen beschränkt, sondern kann auch bei der Neukonzeption vorteilhaft berücksichtigt werden.

Weitere Ausgestaltungen der Erfindung ergeben sich aus den Unteransprüchen. Dabei kann vorgesehen sein, daß als zusätzliches Synthesegas ein Fremd-Synthesegasstrom aus einer getrennten Synthesegasquelle oder ein dem Erdgaseinsatzstrom als Bypass entnommenes, über einen Autothermreformer geleitetes oder aus einer anderen Synthesegaserzeugung stammendes Synthesegas eingesetzt wird.

Das Fremdsynthesegas kann dabei einem kombinierten autothermen Reformer (auch als "CAR" bezeichnet") oder einem Reaktor zur partiellen Oxidation entstammen, der nachgerüstet werden kann und erheblich günstiger baut als die üblicherweise zur Erzeugung des Synthesgases mit exakt passender Gaszusammensetzung verwendeten Apparate. In diesem Fall ergibt sich auch die Möglichkeit, einen Teil des Abgases der Methanol-Erzeugungsanlage, der ansonsten nur noch als Feuerungsgas nutzbar wäre, als weiteres Einsatzgas in einem autothermen Reformer oder einem kombinierten autothermen Reformer mitzuverwenden.

Vorteilhaft kann dabei sein, wenn das den Verlust ausgleichende, dem Haupt-Synthesegasstrom vor der Methanolsynthese zugeführte Zusatz-Synthesegas einem kombinierten Autothermreformer und/oder einem Reaktor zur partiellen Oxidation oder einer anderen Synthesegaserzeugung entnommen wird, wie dies die Erfindung ebenfalls vorsieht.

In einer weiteren Ausgestaltung der Erfindung kann die Abwärme, die bei der Abkühlung des Methanolsynthesegasgemisches aus dem Methanol-Vorreaktor abzuführen ist, dazu genutzt werden, eine Absorptionskältemaschine zu betreiben. Die erzeugte Kälte wird dazu benutzt, das aus den Reformern austretende Synthesegas vor seiner Verdichtung abzukühlen und somit Kompressionsenergie einzusparen.

Die eingesparte Kompressionsenergie kann erfindungsgemäß dazu genutzt werden, das Fremd-Synthesegas bzw. das in nachgerüsteten Reformern erzeugte Zusatz-Synthesegas zu verdichten. Weiterhin kann die erzeugte Kälte dazu genutzt werden, mehr Methanol nach den Methanolreaktoren auszukondensieren. Wenn primär Investitionskosten gespart werden sollen und Energiekosten billig sind, kann es auch wirtschaftlich sein, auf die Abwärmenutzung zu verzichten und statt einer Absorptionskältemaschine eine konventionelle, z.B. mit Ammoniak als Kältemittel betriebene Kältemaschine einzusetzen.

Bei der Mischung des Fremd-Synthesegases mit dem Synthesegas, welches aus dem Methanol-Vorreaktor nach Abscheidung des erzeugten Methanols erhalten wird, ist darauf zu achten, daß die erhaltene Mischung ungefähr der Zusammensetzung des ursprünglich abgezweigten Synthesegases entspricht, die Bedingungen für den vorhandenen Methanolkreislauf voll abdeckt und die ursprüngliche Synthese im Rahmen der Katalysatorbedingungen betrieben werden kann. Um dies zu gewährleisten, muß für die molaren Konzentrationen des Fremd-Synthesegases mindestens gelten: $0,8 \leq \zeta \leq 4$. Eine Verbesserung ergibt sich, wenn außerdem gilt: $1 \leq \zeta \leq 2,5$. Je größer der Anteil des abgezogenen Seitenstroms und des im Vorreaktor erzeugten Methanols ist, desto sorgfältiger ist darauf zu achten, daß die Kennziffer $\zeta$ des Fremd-Synthesegases sich der Zahl 2 nähert.

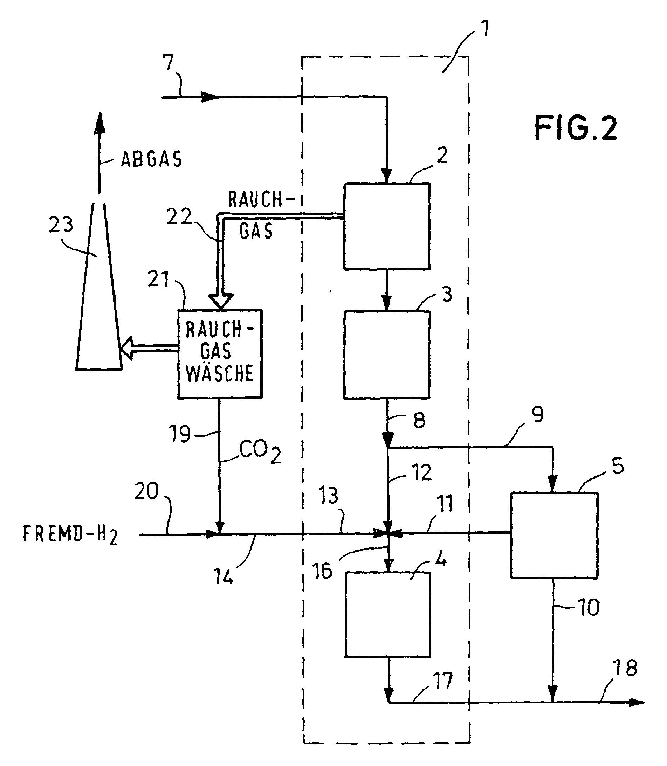

Eine weitere Ausgestaltung der Erfindung besteht darin, daß als zusätzliches Synthesegas eine Mischung aus $H_2$ und $CO_2$ eingesetzt wird, wobei das in der Mischung vorhandene $CO_2$ dem Rauchgas einer Feuerung oder dem Abgas einer $CO_2$-Wäsche in einer Ammoniak-Anlage entstammt (Fig. 2).

Die Nutzung des aus einer Verbrennung stammenden $CO_2$ im zusätzlichen Synthesegas hat den großen Vorteil, daß dieses $CO_2$ nicht in die Atmosphäre abgegeben werden muß, sondern bestimmungsgemäß zur Synthese eingesetzt wird.

In Ausgestaltung ist dabei vorgesehen, daß das $CO_2$ aus einer Rauchgasreinigungsanlage einer Feuerung entstammt, beispielsweise der Feuerung des Primärreformers.

Diese Vorgehensweise hat den zusätzlichen Vorteil, daß das klimawirksame $CO_2$ in den Stoffkreislauf eingebracht wird, so daß beispielsweise eine "Treibhausgas-Emissionsabgabe" nicht mehr hierfür bezahlt werden müßte, was die Verfahrensweise erheblich preiswerter gestaltet.

Neben der Nutzung des $CO_2$ aus einer Verbrennung kann das $CO_2$ auch aus der $CO_2$-Wäsche einer $NH_3$-Anlage entstammen, wobei in der weiteren Beschreibung eine derartige Ammoniak-Anlage nicht näher erläutert wird.

Zur Lösung obiger Aufgabe sieht die Erfindung auch eine Anlage zur Methanolsynthese, insbesondere zur Durchführung des Verfahrens nach einem der vorangehenden Ansprüche vor, mit einer Erdgaszuführleitung zu einem Reformer und einer nachgeschalteten Methanolsynthese, wobei sich eine solche Anlage erfindungsgemäß auszeichnet durch

- eine Abzweigleitung für einen Seitenstrom des den Sekundärreformer verlassenden Synthesegases,
- einen Methanol-Vorreaktor im Seitenstrom,
- eine Methanolzuführleitung zum die Methanolsynthese verlassenden Methanol-Hauptstrom,
- eine nicht umgesetztes Synthesegas aus dem Methanol-Vorreaktor in den Synthesegas-Hauptstrom vor der Methanolsynthese leitende Rückführleitung sowie
- eine Zuführleitung vor der Methanolsynthese zur Zuführung von Verluste ausgleichendem Synthesegas.

In weiterer Ausgestaltung zeichnet sich eine erfindungsgemäße Anlage dadurch aus, daß parallel zum Reformer ein Autothermreformer geschaltet ist, wobei das verlassende Synthesegas wenigstens zum Teil als Verluste ausgleichendes Synthesegas eingesetzt wird.

Nach der Erfindung kann auch eine Rauchgaswäsche für das den Primärreformer verlassende Rauchgas sowie eine $CO_2$-Zuführleitung zur Bereitstellung des Fremd- bzw. Zusatzgases sowie eine Zuführleitung zur Zuführung von Fremd-$H_2$ (Fig. 2) vorgesehen sein, wobei es zweckmäßig sein kann, eine Zuführleitung zur Zuführung von Fremd-$CO_2$ aus einer $CO_2$-Wäsche in der $NH_3$-Anlage sowie eine Zuführleitung zur Zuführung von Fremd-$H_2$ einzusetzen, wie dies in Ausgestaltung nach der Erfindung ebenfalls vorgesehen ist.

Weitere Einzelheiten, Vorteile und Merkmale der Erfindung ergeben sich aufgrund der nachfolgenden Beschreibung sowie anhand der Zeichnung. Diese zeigt in stark vereinfachter Weise in

Fig. 1              ein Blockschaltbild einer erfindungsgemäßen Anlage sowie in den

Fig. 2 und 3      abgewandelte Beispiele des Blockschaltbildes in vergleichbarer Darstellung.

Als Beispiel dient hier die Beschreibung der Nachrüstbarkeit einer Anlage 1, wobei gleichzeitig die Verfahrensfunktionsweise mit beschrieben wird:

Eine bereits in Betrieb befindliche Anlage 1 zur Produktion von Methanol, die aus einem Primärreformer 2, einem Sekundärreformer 3 und einer Methanolsynthese 4 besteht, wird mit einem Methanol-Vorreaktor 5 und einem autothermen Reformer 6 nachgerüstet. Als Einsatzstoff dient entschwefeltes Erdgas 7, das sowohl in den Primärreformer 2 als auch in den autothermen Reformer 6 geleitet wird, wobei noch weitere Einsatzstoffe, die hier nicht dargestellt sind, wie z.B. Wasserdampf und Sauerstoff, hinzukommen. Im Primärreformer 2 wird daraus rohes Synthesegas hergestellt, das im wesentlichen aus Wasserstoff, Kohlenmonoxid, Kohlendioxid und nicht umgesetztem Wasserdampf und Erdgas besteht. Im Sekundärreformer 3 wird mit Sauerstoff das restliche Erdgas zu Wasserstoff und Kohlenmonoxid umgesetzt. Die Bedingungen im Primärreformer 2 und im Sekundärreformer 3 werden dabei so eingestellt, daß ein stöchiometrisches Synthesegas 8 gemäß den Gleichungen (1) bis (4) erzeugt wird. Hiervon wird der Seitenstrom 9 abgezweigt und dem Methanol-Vorreaktor 5 zugeführt. Ein Teil dieses Synthesegases wird im Methanol-Vorreaktor 5 umgesetzt, auskondensiert, abgeschieden und als Zusatz-Methanol 10 abgeführt. Das verbleibende unterstöchiometrische Synthesegas 11 wird dem ursprünglichen Synthesegas-Hauptstrom 12 wieder zugemischt.

Um die Entstehung eines unterstöchiometrischen Gemisches zu vermeiden, wird das überstöchiometrische Synthesegas 13 ebenfalls dem Synthesegas-Hauptstrom 12 zugemischt. Dieses überstöchiometrische Synthesegas 13 besteht seinerseits aus Fremd-Synthesegas 14 und/oder Zusatz-Synthesegas 15, das eigens zu diesem Zweck in dem nachgerüsteten autothermen Reformer 6 erzeugt worden ist, wobei der autotherme Reformer 6 nur als Beispiel zu verstehen ist.

Mit den beiden Zumischungen zum Synthesegas-Hauptstrom 12 wird das stöchiometrische Synthesegas 16 gebildet, welches eine dem stöchiometrischen Synthesegas 8 äquivalente Zusammensetzung aufweist. Damit wird in herkömmlicher Weise in der Alt-Methanolsynthese 4 Methanol 17 erzeugt, das zusammen mit dem Zusatz-Methanol 10 das neue Produkt-Methanol 18 bildet.

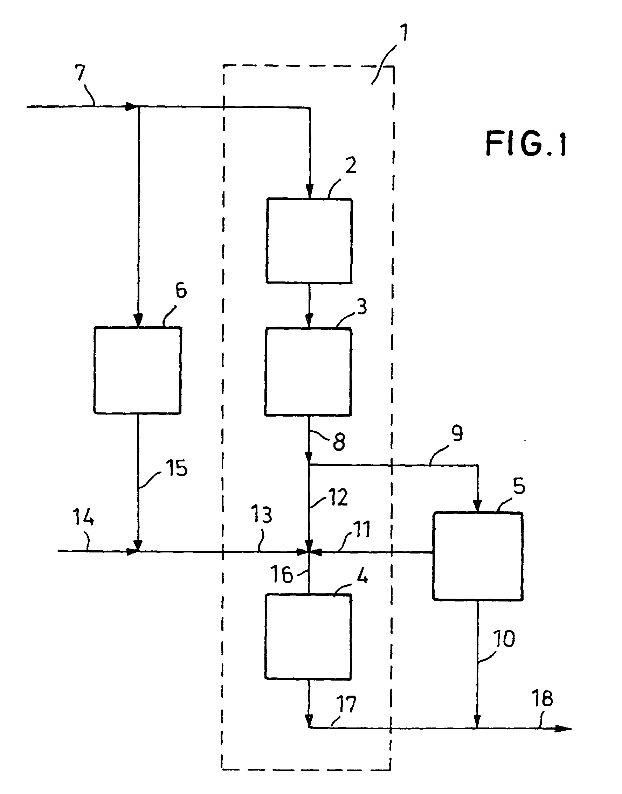

In Abwandlung zur Anlage nach Fig. 1 zeigt Fig. 2 eine Anlage, bei der der Leitung 14, die das Fremdsynthesegas zuführt, über eine Leitung 19 $CO_2$ und über eine Leitung 20 Fremd-$H_2$ zugeführt wird. Das $CO_2$ der Leitung 19 stammt aus einer Rauchgaswäsche 21, die das Rauchgas über die Leitung 22 aus dem Primärreformer 2 zugeführt bekommt. Die Rauchgaswäsche 21 reinigt das Rauchgas, führt das $CO_2$ der Leitung 19 und das Abgas einem nur symbolisch angedeuteten Schornstein 23 zu. Erkennbar ist dieses Abgas $CO_2$-frei.

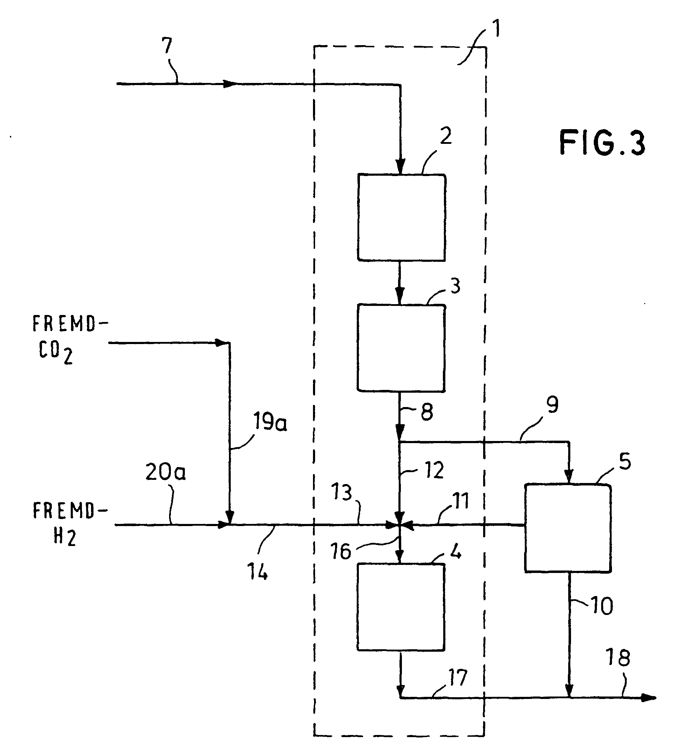

In Fig. 3 ist die Alternative dargestellt, bei der über die Leitung 19a Fremd-$CO_2$ der Leitung 14 für das Fremdsynthesegas zugeführt wird, wobei dieses Fremd-$CO_2$ einer nicht näher dargestellten Ammoniak-Anlage, dort der $CO_2$-Wäsche, entstammt. Auch hier wird Fremd-$H_2$ über eine Leitung 20a dem System zugeführt.

Erkennbar sind insbesondere bei der Variante nach Fig. 2 völlig geschlossene $CO_2$-Kreisläufe möglich, d.h. diese Anlagen arbeiten so, daß kein $CO_2$ der Umwelt zugeführt werden muß.

Der weiteren Veranschaulichung dient das folgende Zahlenbeispiel in Tabelle 1. Hierbei wird zugrundegelegt, daß eine Altanlage zur Produktion von 1000 t/Tag Methanol existiert, die in ihrer Kapazität um rund 35% erweitert werden soll. Die Nummern beziehen sich auf die Bezugszeichen in Fig. 1:

Tabelle 1:

| Nummer | | | 8 | 9 | 10 | 11 | 12 | 13 | 16 | 17 | 18 |
|---|---|---|---|---|---|---|---|---|---|---|---|
| Menge | [kmol/h] | | 5147 | 3119 | 453 | 1730 | 2028 | 1218 | 4975 | 1302 | 1755 |
| | [t/Tag] | | 1405 | 852 | 348 | 491 | 554 | 351 | 1396 | 1000 | 1348 |
| $H_2$ | [kmol/h] | | 3550 | 2151 | 0 | 1215 | 1398 | 832 | 3446 | 0 | 0 |

Tabelle 1: (fortgesetzt)

| Nummer | | 8 | 9 | 10 | 11 | 12 | 13 | 16 | 17 | 18 |
|---|---|---|---|---|---|---|---|---|---|---|
| CO | [kmol/h] | 965 | 585 | 0 | 159 | 380 | 236 | 775 | 0 | 0 |
| $CO_2$ | [kmol/h] | 509 | 309 | 0 | 280 | 201 | 141 | 621 | 0 | 0 |
| $CH_4$ | [kmol/h] | 113 | 69 | 0 | 69 | 45 | 9 | 122 | 0 | 0 |
| $N_2$ | [kmol/h] | 5 | 3 | 0 | 3 | 2 | 2 | 7 | 0 | 0 |
| $H_2O$ | [kmol/h] | 5 | 3 | 0 | 2 | 2 | 0 | 4 | 0 | 0 |
| $\zeta$ | - | 2,064 | 2,064 | | 2,129 | 2,064 | 1,837 | 2,023 | | |
| $CH_3OH$ | [kmol/h] [t/Tag] | 0 | 0 | 453 348 | 1 1 | 0 | 0 | 1 1 | 1302 1000 | 1755 1348 |

## Patentansprüche

1. Verfahren zur Methanolsynthese aus Wasserstoff, Kohlenmonoxid und Kohlendioxid unter Druck, wobei entschwefeltes Erdgas einem Reformer und nachfolgend das Synthesegas einer Methanolsynthese aufgegeben wird, **dadurch gekennzeichnet,**
**daß** nach Durchlaufen des Reformers aus dem Synthesegasstrom ein Seitenstrom einem Methanol-Vorreaktor zugeführt wird, das im Vorreaktor erzeugte Methanol dem die Methanolsynthese des Hauptstromes verlassenden Methanolstrom zugeführt und ein im Methanol-Vorreaktor nicht umgesetzter Synthesegasstrom dem Hauptstrom vor der Methanolsynthese wieder zugeführt wird, wobei im Bereich dieser Zuführung gleichzeitig ein den entstandenen Verlust ausgleichendes zusätzliches Synthesegas aufgegeben wird.

2. Verfahren nach Anspruch 1,
**dadurch gekennzeichnet,**
**daß** als zusätzliches Synthesegas ein Fremd-Synthesegasstrom aus einer getrennten Synthesegasquelle oder ein dem Erdgaseinsatzstrom als Bypass entnommenes, über einen Autothermreformer geleitetes oder aus einer anderen Synthesegaserzeugung stammendes Synthesegas eingesetzt wird.

3. Verfahren nach einem der vorangehenden Ansprüche,
**dadurch gekennzeichnet,**
**daß** das den Verlust ausgleichende, dem Haupt-Synthesegasstrom vor der Methanolsynthese zugeführte Zusatz-Synthesegas einem kombinierten Autothermreformer und/oder einem Reaktor zur partiellen Oxidation oder einer anderen Synthesegaserzeugung entnommen wird.

4. Verfahren nach einem der vorangegangenen Ansprüche,
**dadurch gekennzeichnet,**
**daß** die Abwärme des zusätzlichen Methanol-Vorreaktors zum Betrieb einer Absorptionskältemaschine eingesetzt wird, wobei die erzeugte Kälte zur Kühlung einer Synthesegaskompression eingesetzt wird und/oder nicht genutzte Kompressionsenergie zur Verdichtung von Fremdsynthesegas eingesetzt wird.

5. Verfahren nach einem der vorangehenden Ansprüche,
**dadurch gekennzeichnet,**
**daß** als zusätzliches Synthesegas eine Mischung aus $H_2$ und $CO_2$ eingesetzt wird, wobei das in der Mischung vorhandene $CO_2$ dem Rauchgas einer Feuerung oder dem Abgas einer $CO_2$-Wäsche in einer Ammoniak-Anlage entstammt.

6. Verfahren nach einem der vorangehenden Ansprüche,
**dadurch gekennzeichnet,**
**daß** das $CO_2$ aus einer Rauchgasreinigungsanlage einer Feuerung entstammt, beispielsweise der Feuerung des Primärreformers.

7. Anlage zur Methanolsynthese mit einer Erdgaszuführleitung zu einem Reformer und einer nachgeschalteten Methanolsynthese, **gekennzeichnet durch**

- eine Abzweigleitung (9) für einen Seitenstrom des den Sekundärreformer (3) verlassenden Synthesegases (8),
- einen Methanol-Vorreaktor (5) im Seitenstrom,
- eine Methanolzuführleitung (10) zum die Methanolsynthese (4) verlassenden Methanol-Hauptstrom (17),
- eine nicht umgesetztes Synthesegas aus dem Methanol-Vorreaktor (5) in den Synthesegas-Hauptstrom (12) vor der Methanolsynthese (4) leitende Rückführleitung (11) sowie
- eine Zuführleitung (13) vor der Methanolsynthese (4) zur Zuführung von Verluste ausgleichendem Synthesegas.

**8.** Anlage nach Anspruch 7,
**dadurch gekennzeichnet,**
**daß** parallel zum Reformer (2,3) ein Autothermreformer (6) geschaltet ist, wobei das verlassende Synthesegas (15) wenigstens zum Teil als Verluste ausgleichendes Synthesegas eingesetzt wird.

**9.** Anlage nach Anspruch 7 oder 8,
**gekennzeichnet durch**
eine Rauchgaswäsche (21) für das den Primärreformer (2) verlassende Rauchgas sowie einer $CO_2$-Zuführleitung (19) zur Bereitstellung des Fremd- bzw. Zusatzsynthesegases (14) sowie einer Zuführleitung (20) zur Zuführung von Fremd-$H_2$ (Fig. 2).

**10.** Anlage nach Anspruch 7 oder einem der folgenden, **gekennzeichnet durch**
eine Zuführleitung (19a) zur Zuführung von Fremd-$CO_2$ aus einer $CO_2$-Wäsche in der $NH_3$-Anlage sowie einer Zuführleitung (20c) zur Zuführung von Fremd-$H_2$ (Fig. 3).

**Claims**

**1.** Method for the synthesis of methanol from hydrogen, carbon monoxide and carbon dioxide under pressure, wherein desulphurated natural gas is sent forward to a reformer and subsequently the synthesis gas is sent forward to methanol synthesis,
**characterised in that**
after passing through the reformer, out of the stream of synthesis gas a side stream is supplied to a methanol pre-reactor, the methanol created in the pre-reactor is supplied to the methanol stream leaving the methanol synthesis of the main stream, and a synthesis gas stream that is not converted in the methanol pre-reactor is supplied once more to the main stream before the methanol synthesis, wherein in the area of this supply, at the same time an additional synthesis gas is given up which compensates the loss that has arisen.

**2.** Method in accordance with claim 1, **characterised in that** as an additional synthesis gas, a foreign synthesis gas stream from a separate synthesis gas source is used, or a synthesis gas that has been extracted from the natural gas utilisation stream as a bypass, led via an autotherm reformer, or originating from another synthesis gas production.

**3.** Method in accordance with one of the preceding claims, **characterised in that** the additional synthesis gas, which compensates the loss and is supplied to the main synthesis gas stream before the methanol synthesis, is taken from a combined autotherm reformer and/or a reactor for partial oxidation, or [from] another synthesis gas production.

**4.** Method in accordance with one of the preceding claims, **characterised in that** the waste heat of the additional methanol pre-reactor is used to operate an absorption-type refrigerating machine, wherein the cold that is created is used to cool a synthesis gas compression and/not unused compression energy is used for the compression of foreign synthesis gas.

**5.** Method in accordance with one of the preceding claims, **characterised in that** as additional synthesis gas, a mixture of $H_2$ and $CO_2$ is used, wherein the $CO_2$ that is present in the mixture originates from the flue gas of a firing place or the waste gas of $CO_2$ washer in an ammonia plant.

**6.** Method in accordance with one of the preceding claims, **characterised in that** the $CO_2$ originates from a flue gas scrubbing plant of a firing place, for example the firing place of the primary reformer.

7. Plant for methanol synthesis with a natural gas supply conduit to a reformer and a downstream methanol synthesis, **characterised by**

   - branch conduit (9) for a side stream of the synthesis gas (8) leaving the secondary reformer (3),
   - a methanol pre-reactor (5) in the side stream,
   - a methanol supply conduit (10) to the methanol main stream (17) leaving the methanol synthesis (4),
   - a return conduit (11) which conducts non-converted synthesis gas from the methanol pre-reactor (5) into the synthesis gas main stream (12) before the methanol synthesis (4), as well as
   - a supply conduit (13) before the methanol synthesis (4), for the supply of synthesis gas that compensates losses.

8. Plant in accordance with claim 7, **characterised in that** switched parallel to the reformer (2, 3) is an autotherm reformer (6), wherein the synthesis gas (15) that is leaving is used at least in part as synthesis gas for compensating losses.

9. Plant in accordance with claim 7 or 8, **characterised by** a flue gas scrubbing plant (21) for the flue gas leaving the primary reformer (2), as well as a $CO_2$ supply conduit (19) for providing the foreign or additional synthesis gas (14), as well as a supply conduit (20) for supplying foreign $H_2$ (fig. 2).

10. Plant in accordance with claim 7 or one of the following ones, **characterised by** a supply conduit (19a) for supplying foreign $CO_2$ from a $CO_2$ scrubbing plant in the $NH_3$ plant, as well as a supply conduit (20c) for supplying foreign $H_2$ (fig. 3).

**Revendications**

1. Procédé de synthèse du méthanol à partir d'hydrogène, de monoxyde de carbone et dioxyde de carbone sous pression, dans lequel du gaz naturel désulfuré alimente un reformeur et le gaz de synthèse alimente à son tour une synthèse de méthanol
   **caractérisé en ce que**
   à partir du flux de gaz de synthèse, suite au passage dans le reformeur, une fraction latérale est introduite dans un réacteur préliminaire pour le méthanol, **en ce que** le méthanol produit dans le réacteur préliminaire alimente le flux de méthanol sortant de la synthèse du méthanol du flux principal et **en ce qu'**un flux de gaz de synthèse non transformé dans le réacteur préliminaire pour le méthanol est réintroduit dans le flux principal préalablement à la synthèse du méthanol, où l'on ajoute simultanément, dans la zone de cette introduction, un gaz de synthèse supplémentaire qui compense la déperdition occasionnée.

2. Procédé selon la revendication 1
   **caractérisé en ce que**
   l'on met en oeuvre en tant que gaz de synthèse supplémentaire un flux de gaz de synthèse étranger provenant d'une source de gaz de synthèse distincte ou un gaz de synthèse prélevé en dérivation d'un flux de charge de gaz naturel, conduit à travers un reformeur autotherme ou provenant d'une autre production de gaz de synthèse.

3. Procédé selon l'une quelconque des revendications précédentes
   **caractérisé en ce que**
   le gaz de synthèse en supplément qui compense la déperdition et alimente le flux de gaz de synthèse principal préalablement à la synthèse du méthanol est retiré d'un reformeur autotherme combiné et/ou d'un réacteur pour oxydation partielle ou d'une autre production de gaz de synthèse.

4. Procédé selon l'une quelconque des revendications précédentes
   **caractérisé en ce que**
   l'on emploie la chaleur dissipée du réacteur préliminaire supplémentaire pour le méthanol pour faire fonctionner une machine frigorifique à absorption, où le froid produit est employé pour le refroidissement d'une compression du gaz de synthèse et/ou de l'énergie de compression non utilisée est employée pour la condensation du gaz de synthèse étranger.

5. Procédé selon l'une quelconque des revendications précédentes
   **caractérisé en ce que**

l'on emploie en tant que gaz de synthèse supplémentaire un mélange de $H_2$ et de $CO_2$, où le $CO_2$ présent dans le mélange provient du gaz de fumée d'une combustion ou du gaz perdu d'un lavage $CO_2$ dans une installation pour l'ammoniac.

6. Procédé selon l'une quelconque des revendications précédentes
   **caractérisé en ce que**
   le $CO_2$ provient d'une installation d'épuration de gaz de fumée d'une combustion, par exemple de la combustion du reformeur primaire.

7. Installation de synthèse du méthanol dotée d'une conduite d'amenée de gaz naturel à un reformeur et d'une synthèse de méthanol reliée en aval,
   **caractérisée par** :

   - une dérivation (9) pour une fraction latérale du gaz de synthèse (8) sortant du reformeur secondaire (3),
   - un réacteur préliminaire pour méthanol (5) au niveau de la fraction latérale,
   - une conduite d'amenée de méthanol (10) au flux principal de méthanol (17) sortant de la synthèse du méthanol (4),
   - une conduite de recyclage (11) acheminant du gaz de synthèse non transformé du réacteur préliminaire pour le méthanol (5) au flux principal de gaz de synthèse (12) en amont de la synthèse du méthanol (4) et
   - une conduite d'amenée (13) en amont de la synthèse du méthanol (4) pour introduire du gaz de synthèse qui compense les déperditions.

8. Installation selon la revendication 7
   **caractérisée en ce qu'un**
   reformeur autotherme (6) est monté parallèlement au reformeur (2, 3), où le gaz de synthèse sortant (15) est au moins en partie employé en tant que gaz de synthèse compensant les déperditions.

9. Installation selon la revendication 7 ou 8,
   **caractérisée par**
   un lavage de gaz de fumée (21) pour le gaz de fumée sortant du reformeur primaire (2) ainsi qu'une conduite d'amenée de $CO_2$ (19) servant à la préparation du gaz de synthèse en supplément ou étranger (14) ainsi qu'une conduite d'amenée (20) servant à introduire du $H_2$ étranger (fig. 2).

10. Installation selon la revendication 7 ou l'une des suivantes
    **caractérisée par**
    une conduite d'amenée (19a) servant à acheminer du $CO_2$ étranger d'un lavage $CO_2$ à l'installation pour $NH_3$ ainsi qu'une conduite d'amenée (20c) pour l'alimentation en $H_2$ étranger (fig. 3).

FIG.1

FIG.2

ABGAS

RAUCH-
GAS

RAUCH-
GAS
WÄSCHE

$CO_2$

FREMD-$H_2$

10

FIG.3